(19) Europäisches Patentamt
European Patent Office
Office européen des brevets

(11) EP 1 156 805 B1

(12) **EUROPÄISCHE PATENTSCHRIFT**

(45) Veröffentlichungstag und Bekanntmachung des Hinweises auf die Patenterteilung:
**11.08.2004 Patentblatt 2004/33**

(21) Anmeldenummer: **00902659.2**

(22) Anmeldetag: **05.02.2000**

(51) Int Cl.$^7$: **A61K 31/428**, A61P 3/04

(86) Internationale Anmeldenummer:
**PCT/EP2000/000928**

(87) Internationale Veröffentlichungsnummer:
**WO 2000/051579 (08.09.2000 Gazette 2000/36)**

(54) **VERWENDUNG VON POLYZYKLISCHEN THIAZOL-SYSTEMEN ZUR HERSTELLUNG VON MEDIKAMENTEN ZUR PROPHYLAXE ODER BEHANDLUNG VON OBESITAS**

USE OF POLYCYCLIC THIAZOLE SYSTEMS FOR MANUFACTURING MEDICAMENTS FOR PREVENTING OR TREATING OBESITY

UTILISATION DE SYSTEMES DE THIAZOL POLYCYCLIQUES POUR LA FABRICATION DE MEDICAMENTS SERVANT A LA PROPHYLAXIE OU AU TRAITEMENT DE L'OBESITE

(84) Benannte Vertragsstaaten:
**AT BE CH CY DE DK ES FI FR GB GR IE IT LI LU NL PT SE**

(30) Priorität: **26.02.1999 DE 19908535**

(43) Veröffentlichungstag der Anmeldung:
**28.11.2001 Patentblatt 2001/48**

(73) Patentinhaber: **Aventis Pharma Deutschland GmbH**
**65929 Frankfurt am Main (DE)**

(72) Erfinder:
- **JÄHNE, Gerhard**
**D-65929 Frankfurt (DE)**
- **GEISEN, Karl**
**D-60318 Frankfurt (DE)**
- **LANG, Hans-Jochen**
**D-65719 Hofheim (DE)**
- **BICKEL, Martin**
**D-61348 Bad Homburg (DE)**

(56) Entgegenhaltungen:
**EP-A- 0 666 263**
**EP-A- 0 749 966**
**DE-A- 19 908 533**
**US-A- 5 360 803**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 156 805 B1

Printed by Jouve, 75001 PARIS (FR)

## Beschreibung

Die Erfindung betrifft die Verwendung polycyclischer Thiazol-Systeme sowie derer physiologisch verträgliche Salze und physiologisch funktionellen Derivate zur Herstellung von Medikamenten zur Prophylaxe oder Behandlung von Obesitas.

In EP 0 749 966 sind polycyclische Thiazolsysteme mit 5-$HT_3$-Rezeptor agonistischen Eigenschaften als Wirkstoffe für die Behandlung von ZNS-Störungen beschrieben.

Der Erfindung lag die Aufgabe zugrunde, Verbindungen zur Verfügung zu stellen, die eine therapeutisch verwertbare anorektische Wirkung entfalten.

Die Erfindung betrifft daher die Verwendung der Verbindungen der Formel 1,

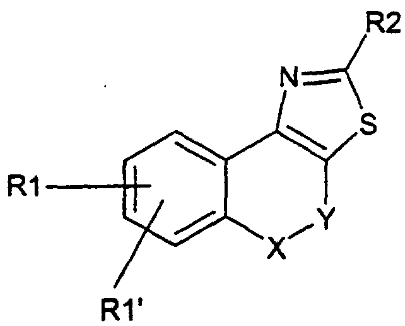

I

worin bedeuten

Y $CH_2$, $CH_2$-$CH_2$;

X eine direkte Bindung, $CH_2$, O, NR3 oder S;

R1, R1' unabhängig voneinander H, F, Cl, Br, J, $CF_3$, CN, COOH, COO($C_1$-$C_6$)Alkyl, $CONH_2$, CONH($C_1$-$C_6$)Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, $OCF_3$, O-($C_2$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, CN, OC(O)$CH_3$, OC(O)H, O-$CH_2$-Ph, $NH_2$, NH-CO-$CH_3$ oder N ($COOCH_2Ph$)$_2$ ersetzt sein kann;
$SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Phenyl, $SO_2$-($C_1$-$C_6$)Alkyl, SO-($CH_2$)$_n$-Phenyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$)-Alkyl, ($C_1$-$C_6$)-Alkyl, $NH_2$ substituiert sein kann; $NH_2$, NH-($C_1$-$C_6$)-Alkyl, N(($C_1$-$C_6$)-Alkyl)$_2$, NH($C_1$-$C_7$)-Acyl, Phenyl, Biphenylyl, O-($CH_2$)$_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;

R2 ($C_1$-$C_8$)-Alkyl, ($C_3$-$C_7$)-Cycloalkyl, ($C_2$-$C_6$)-Alkenyl, ($C_2$-$C_6$)-Alkinyl, C(CN)=C($CH_3$)$_2$, C(O)$OCH_2CH_3$, $CH_2$-O-C(O)-C($CH_3$)$_3$,
($C_4$-$C_7$)-Cycloalkenyl, wobei in den Alkylresten ein, mehrere, oder alle Wasserstoff(e) durch Fluor ersetzt sein können oder ein Wasserstoff durch OH, CN, oder O-($C_1$-$C_4$)-Alkyl ersetzt sein kann;
($CH_2$)$_n$-NR6R7, wobei n = 1-6 sein kann und R6 und R7 unabhängig voneinander H, ($C_1$-$C_6$)-Alkyl, ($C_3$-$C_6$) -Cycloalkyl, CO-($C_1$-$C_6$)-Alkyl, CHO oder CO-Phenyl sein können, oder-NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
($CH_2$)$_n$-Aryl, wobei n = 0 - 6 sein kann und Aryl Phenyl, Biphenylyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3-oder 5-Isoxazolyl, 2- oder 3-Pyrrolyl, 2- oder 3-Pyridazinyl, 2-, 4- oder 5-Pyrimidinyl, 2-Pyrazinyl, (1,3,5-Triazin)-2, 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, $CF_3$, $NO_2$, CN, $OCF_3$, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]$_2$, CONH($C_3$-$C_6$)Cycloalkyl, $NH_2$, NH-CO-($C_1$-$C_6$)-Alkyl, NH-CO-Phenyl, Pyrrolidin-1-yl, Morpholin-1-yl, Piperidin-1-yl, Piperazin-1-yl, 4-Methyl-piperazin-1-yl, $(CH_2)_n$-Phenyl, O-$(CH_2)_n$-Phenyl, S-$(CH_2)_n$-Phenyl, $SO_2$-$(CH_2)_n$-Phenyl, wobei n = 0-3, substituiert sein kann;

R3 H, ($C_1$-$C_6$)-Alkyl; CO-($C_1$-$C_5$)-Alkyl, CO-($C_3$-$C_6$)-cycloalkyl; $SO_2$-phenyl, p-Toluolsulfonyl;

sowie derer physiologisch verträgliche Salze zur Herstellung eines Medikaments zur Prophylaxe oder Behandlung von Obesitas.

Bevorzugt ist die Verwendung der Verbindungen der Formel I, in denen ein oder mehrere Rest(e) die folgende Bedeutung hat bzw. haben:

Y $CH_2$;

X eine direkte Bindung, $CH_2$;

R1, R1' unabhängig voneinander H, F, Cl, Br, J, $CF_3$, CN, COOH, COO($C_1$-$C_6$)Alkyl, $CONH_2$, CONH($C_1$-$C_6$)Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, $OCF_3$, O-($C_2$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, CN, $NH_2$, ersetzt sein kann;
$NH_2$, NH-($C_1$-$C_6$)-Alkyl, N(($C_1$-$C_6$)-Alkyl)$_2$, Phenyl, O-$(CH_2)_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R2 ($C_1$-$C_8$)-Alkyl, ($C_3$-$C_7$)-Cycloalkyl, ($C_2$-$C_6$)-Alkenyl, ($C_2$-$C_6$)-Alkinyl, C(CN)=C$(CH_3)_2$, C(O)$OCH_2CH_3$, $CH_2$-O-C(O)-C$(CH_3)_3$, ($C_4$-$C_7$)-Cycloalkenyl, wobei in den Alkylresten ein, mehrere, oder alle Wasserstoff(e) durch Fluor ersetzt sein können oder ein Wasserstoff durch OH, CN, oder O-($C_1$-$C_4$)-Alkyl ersetzt sein kann;
$(CH_2)_n$-NR6R7, wobei n = 1-6 sein kann und R6 und R7 unabhängig voneinander H, ($C_1$-$C_6$)-Alkyl, ($C_3$-$C_6$) -Cycloalkyl, CO-($C_1$-$C_6$)-Alkyl, CHO oder CO-Phenyl sein können;
$(CH_2)_n$-Aryl, wobei n = 0 - 6 sein kann und Aryl Phenyl, Biphenylyl, 1- oder 2-Naphthyl, 2-, 3- oder 4-Pyridyl, Benzothiazol-2-yl, Indol-3-yl, Indol-5-yl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

sowie derer physiologisch verträgliche Salze zur Herstellung eines Medikaments zur Prophylaxe oder Behandlung von Obesitas

Besonders bevorzugt ist die Verwendung von Verbindungen der Formel 1, in denen ein oder mehrere Rest (e) die folgende Bedeutung hat bzw. haben:

Y -$CH_2$-;

X -$CH_2$-, eine direkte Bindung;

R1 Cl, Br, ($C_1$-$C_6$)-Alkyl, $OCF_3$, O-($C_2$-$C_6$)-Alkyl, wobei in den Alkylresten ein, mehrere, oder alle Wasserstoff(e) durch Fluor ersetzt sein können;
Phenyl, das bis zu 3-fach substituiert sein kann mit F, Cl, Br, OH, ($C_1$-$C_6$)-Alkyl;

R1' H oder R1;

R2 ($C_1$-$C_6$)-Alkyl, ($C_1$-$C_6$)-Alkyl-CN, C(CN)=C$(CH_3)_2$, C(O)$OCH_2CH_3$, $CH_2$-O-C(O)-C$(CH_3)_3$;

$(CH_2)_n$-Aryl, wobei n = 0 - 2 sein kann und Aryl gleich Phenyl, 2-, 3- oder 4-Pyridyl, Benzothiazol-2-yl, Indol-3-yl, Indol-5-yl sein kann und der Arylrest oder Heteroarylrest bis zu zweifach mit F, Cl, Br, OH, $CF_3$, $NO_2$, CN, $OCF_3$, $(C_1-C_6)$-Alkyl, O-$(C_1-C_6)$-Alkyl, substituiert sein kann;

sowie derer physiologisch verträgliche Salze zur Herstellung eines Medikaments zur Prophylaxe oder Behandlung von Obesitas.

Die Erfindung bezieht sich auf Verwendung der Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R1', R2 und R3 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorid verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (1) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge

der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die folgenden Zubereitungen dienen zur Erläuterung der Erfindung ohne diese jedoch einzuschränken.

Beispiel A

Gelatineweichkapseln, enthaltend 100 mg Wirkstoff pro Kapsel:

| | pro Kapsel |
|---|---|
| Wirkstoff | 100 mg |
| aus Kokosfett fraktioniertes | |
| Triglycerid-Gemisch | 400 mg |
| Kapselinhalt | 500 mg |

Beispiel B

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel C

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

Beispiel E

Dragee, enthaltend 50 mg Wirkstoff pro Dragee:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

Beispiel F

Für die Herstellung des Inhalts von Hartgelatinekapseln eignen sich die folgenden Rezepturen:

| | |
|---|---|
| a) Wirkstoff | 100 mg |
| Maisstärke | 300 mg |
| | 400 mg |
| b) Wirkstoff | 140 mg |
| Milchzucker | 180 mg |

(fortgesetzt)

| | |
|---|---|
| Maisstärke | 180 mg |
| | 500 mg |

Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entimineralisiertes Wasser | ad 100 ml |

Gegenstand der Erfindung ist weiterhin ein Verfahren zur Herstellung der Verbindungen der allgemeinen Formel 1, dadurch gekennzeichnet, daß man Verbindungen der allgemeinen Formel 1 gemäß folgendem Reaktionsschema darstellt:

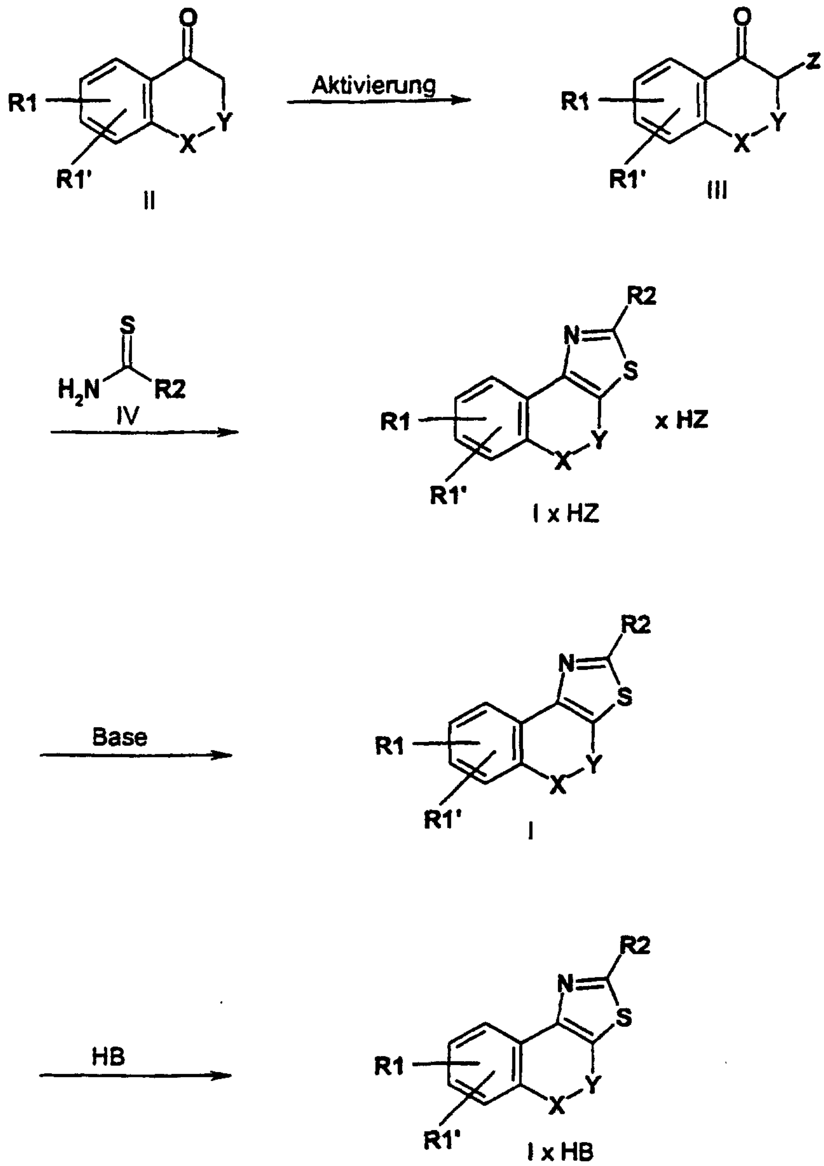


II

III

I x HZ

I

I x HB

Bicyclische Ketone der allgemeinen Formel II, worin R1, R1', X und Y die angegebenen Bedeutungen besitzen, sind entweder kommerziell erhältlich oder nach literaturbekannten Methoden herstellbar.

Bicyclische Ketone der Formel II, worin R1 oder R1' Arylreste darstellen, können durch Pd(O)-katalysierte Addition von Boronsäureestem an Verbindungen der Formel II, worin R1 und/oder R1' Brom, Jod oder Trifluormethylsulfonyloxy darstellen, gewonnen werden (z.B.: N. Miyaura und A. Suzuki, Chem. Rev. 95, 2457-83 (1995) oder T. Ohe, N. Miyaura und A. Suzuki, J. Org. Chem. 58, 2201-08 (1993)).

Bicyclische Ketone der allgemeinen Formel II, worin R1 und/oder R1' Alkinylreste oder Alkenylreste darstellen, können z.B. mit Methoden wie sie bei K. Sonagashira et al., Tetrahedron Lett. 4467 (1975) und S. Takahashi et al., Synthesis 627 (1980) (palladiumkatalysierte Umsetzung von z. B. Trimethylsilylacetylen oder Alkinen) oder bei E. Negishi et al., J. Org. Chem. 62, 8957-60 (1997) (Alkinylzinkbromide) oder bei A. Hassner et al., J. Org. Chem. 49, 2546 (1984) (Trialkylzinnalkine, Trialkylzinnvinyl- oder allylverbindungen, 1-Alkenylborverbindungen oder Vinylverbindungen) beschrieben sind, hergestellt werden.

Die Aktivierung der bicyclischen Ketone der allgemeinen Formel II erfolgt am einfachsten durch Umsetzung mit Brom zum alpha-Bromketon der allgemeinen Formel III (Z = Br). Z in den aktivierten Verbindungen der allgemeinen Formel III kann jedoch auch vorteilhaft Cl, J. $O\text{-}C(O)C_6H_4\text{-}NO_2$, $O\text{-}SO_2CH_3$, $O\text{-}SO_2CF_3$, $O\text{-}SO_2\text{-}C_6H_4\text{-}4\text{-}CH_3$ oder $O\text{-}SO_2\text{-}C_6H_5$ sein.

Verbindungen der allgemeinen Formel I xHZ werden gewonnen, indem man Thioamide der allgemeinen Formel IV, worin R2 die angegebenen Bedeutungen besitzt, umsetzt. Dabei wird vorteilhaft so verfahren, daß man die Verbindungen III mit den Thioamiden IV im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen, Grundsätzlich können die Reaktionspartner auch ohne Lösemittel umgesetzt werden, insbesondere dann, wenn das jeweilige Thioamid einen tiefen Schmelzpunkt hat. Die Reaktion verläuft nur wenig exotherm und kann zwischen - 10°C und 150°C. bevorzugt zwischen 50°C und 100°C, durchgeführt werden. Als besonders günstig erweist sich in der Regel ein Temperaturbereich zwischen 50°C und 80°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Die erhaltenen Salze der Verbindungen der allgemeinen Formel I x HZ lassen sich mit organischen oder anorganischen Basen in die freien basischen Verbindungen der Formel I überführen.

Die Verbindungen der allgemeinen Formel I lassen sich durch Umsatz mit organischen oder anorganischen Säuren der Formel HB in ihre Säureadditionssalze der allgemeinen Formel I $\times$ HB überführen.Als anorganische Säuren HB kommen beispielsweise in Betracht: Halogenwasserstoffsäuren wie Chlorwasserstoffsäure und Bromwasserstoffsäure, sowie Schwefelsäure, Phosphorsäure und Amidosulfonsäure. Als organische Säuren HB seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Erfindungsgemäß wurden außer den in den Ausführungsbeispielen beschriebenen Derivaten auch die in den folgenden Tabellen zusammengestellten Verbindungen der allgemeinen Formel I bzw. deren Säureadditionsprodukte erhalten:

Tabelle 1: Beispiele

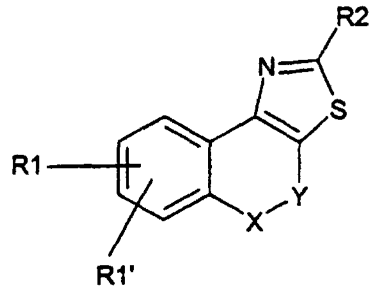

Formel I

| Beispiel | $R_1$; $R_1$' | $R_2$ | X | Y | Salz | Fp. [°C] |
|---|---|---|---|---|---|---|
| 1 | 6-Cl; H | $CH_3$ | - | $CH_2$ | HCl | 227 |
| 2 | 7-($C_6H_4$-4-$CF_3$) ; H | $CH_3$ | - | $CH_2$ | - | 137 |
| 3 | 5-($C_6H_4$-4-$CF_3$) ; H | $CH_3$ | - | $CH_2$ | - | 159 |
| 4 | 6-Cl; H | $C(CN)=C(CH_3)_2$ | - | $CH_2$ | - | Z. ab 132 |
| 5 | 6-Cl; H | $C(O)OCH_2CH_3$ | - | $CH_2$ | - | 162 |
| 6 | 6-Cl; H | $CH_2$-CN | - | $CH_2$ | - | 134 |
| 7 | 6-Cl; H | $CH_3$ | - | $CH_2$ | - | Z. ab 125 |
| 8 | 6-Cl; H | $C_6H_4$-4-$CF_3$ | - | $CH_2$ | - | 130 |
| 9 | 6-Cl; H | $C_6H_5$ | - | $CH_2$ | - | 106 |
| 10 | 6-($C_6H_4$-4-Cl) ; H | $CH_3$ | - | $CH_2$ | - | 173 |
| 11 | 6-($C_6H_4$-3-Cl) ; H | $CH_3$ | - | $CH_2$ | - | 112 |
| 12 | 6-Cl; H | $CH_2$-O-C(O)-$C(CH_3)_3$ | - | $CH_2$ | - | 105 |
| 13 | 6-O-$CH_2$-$CF_3$; H | $CH_3$ | - | $CH_2$ | HBr | 222 |
| 14 | 6-O-$CH_2$-$CF_3$; H | $C_6H_5$ | - | $CH_2$ | HBr | 252 |
| 15 | 6-O-$C_6H_4$-4-Cl; H | $CH_3$ | - | $CH_2$ | HBr | 234 |
| 16 | 6-O-$C_6H_4$-4-Cl; H | $C_6H_5$ | - | $CH_2$ | HBr | 253 |
| 17 | 6-O-$C_6H_4$-3-$CH_3$; H | $C_6H_5$ | - | $CH_2$ | HBr | 271 |
| 18 | 6-O-$C_6H_4$-3-$CH_3$; H | $CH_3$ | - | $CH_2$ | HBr | 240 |

| | | | | | | |
|---|---|---|---|---|---|---|
| 19 | 5-Br; H | $C_6H_5$ | $CH_2$ | $CH_2$ | - | 85 |
| 20 | 5,6-di-$CH_3$; H | $CH_3$ | - | $CH_2$ | HBr | 277 |
| 21 | 5,6-di-$CH_3$; H | $C_6H_5$ | - | $CH_2$ | HBr | 290 |
| 22 | 6-Cl; H | $C_6H_4$-4-OH | - | $CH_2$ | - | 280 |
| 23 | 6-Cl; H | $C_6H_4$-2-OH | - | $CH_2$ | - | 198 |
| 24 | 6-Cl; H | Pyrid-3-yl | - | $CH_2$ | - | 169 |
| 25 | 6-Cl; H | $CH_2$-Indol-3-yl | - | $CH_2$ | - | 186 |
| 26 | 6-Cl; H | $CH_2$-benzthiazol-2-yl | - | $CH_2$ | - | 126 |
| 27 | H; H | $CH_3$ | - | $CH_2$ | HCl | 233 |

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Wirksamkeit der Verbindungen wurde wie folgt getestet:

Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

Tabelle 2: Anorektische Wirkung, gemessen als Reduktion des kumulierten Milchkonsums behandelter im Vergleich zu dem unbehandelter Tiere.

| Verbindung/Beispiel<br>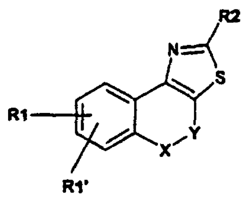<br>Formel I | Orale Dosis<br><br>[mg/kg] | Anzahl der Tiere / Kumulierter Milchkonsum der behandelten Tiere<br><br>N / [ml] | Anzahl der Tiere / Kumulierter Milchkonsum der unbehandelten Kontrolltiere<br><br>N / [ml] | Reduktion des kumulierten Milchkonsums in % der Kontrolle |
|---|---|---|---|---|
| Beispiel 5 | 50 | 5 / 2,26 | 5 / 4,02 | 44 |
| Beispiel 6 | 50 | 5 / 2,28 | 5 / 4,02 | 43 |
| Beispiel 7 | 50 | 5 / 0,58 | 5 / 3,44 | 83 |
| Beispiel 11 | 50 | 4 / 1,58 | 5 / 3,24 | 51 |
| Beispiel 13 | 50 | 5 / 1,82 | 5 / 3,80 | 52 |
| Beispiel 20 | 50 | 5 / 1,98 | 5 / 4,06 | 51 |

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

Ausführungsbeispiel 1:

6-Chlor-2-methyl-8H-indeno[1,2-d]thiazol Hydrochlorid (Verbindung des Beispiels 1):

a) 2-Brom-5-chlor-indan-1-on:

5-Chlor-indan-1-on wird mit Brom in Eisessig unter Einsatz einer katalytischen Menge 48%iger HBr-Lösung in Wasser bei Raumtemperatur umgesetzt. Man erhält 2-Brom-5-chfor-indan-1-on mit dem Schmelzpunkt 94-96°C.

b) 6-Chlor-2-methyl-8H-indeno[1,2-d]thiazol Hydrochlorid:

1g 2-Brom-5-chlor-indan-1-on werden mit 0.31 g Thioacetamid in 100 ml Aceton 5 h bei Raumtemperatur gerührt. Der Niederschlag wird abgesaugt, mit Aceton gewaschen und im Vakuum getrocknet. Das Hydrobromid wird in Essigester suspendiert, mit Triethylamin neutralisiert und die Essigesterlösung wird mit Wasser gewaschen, über Natriumsulfat getrocknet, filtriert und im Vakuum eingeengt. Die freie Base wird in Tetrahydrofuran gelöst und die Lösung mit einem Überschuß etherischer HCl versetzt. Die Mischung wird 2 h bei Raumtemperatur gerührt und dann im Vakuum eingeengt Der Rückstand (Hydrochlorid des 6-Chlor-2-methyl-8,8a-dihydro-indeno[1,2-d]thiazol-3a-ols) wird in 30 ml Eisessig 4 h unter Rückfluß erhitzt. Das Lösemittel wird im Vakuum entfernt, der Rückstand mit Diisopropylether verrührt und man erhält 6-Chlor-2-methyl-8Hindeno[1,2-d]thiazol Hydrochlorid mit dem Schmelzpunkt 135°C.

Ausführungsbeispiel 2:

(6-Chlor-8H-indeno[1,2-d]thiazol-2-yl)-acetonitril (Verbindung des Beispiels 6):

1 g (4 mmol) 2-Brom-5-chlor-indan-1-on werden mit 450 mg (4.5 mmol) 2-Cyanothioacetamid und 0.55 ml (4 mmol) Triethylamin in 10 ml trockenem Ethanol 4 h bei Raumtemperatur gerührt. Das Reaktionsgemisch wird im Vakuum eingeengt, und der Rückstand wird an Kieselgel mit Essigsäureethylester / n-Heptan 1 /1 chromatographisch gereinigt. Man erhält (6-Chlor-8H-indeno[1,2-d]thiazol-2-yl)-acetonitril mit dem Schmelzpunkt 134°C.

Ausführungsbeispiel 3:

6-Chlor-2-methyl-8H-indeno[1,2-d]thiazol (Verbindung des Beispiels 7):

Eine Suspension der Verbindung des Ausführungsbeispieles 1 in Essigsäureethylester wird mit einer konzentrierten wässrigen Natriumhydrogencarbonatlösung mehrmals ausgeschüttelt; die Essigesterphase wird anschließend über Natriumsulfat getrocknet, filtriert und im Vakuum eingeengt. Man erhält 6-Chlor-2-methyl-8H-indeno[1,2-d] thiazol mit dem Schmelzpunkt 94°C.

Ausführungsbeispiel 4:

6-(3-Chlor-phenyl)-2-methyl-8H-indeno[1,2-d]thiazol (Verbindung des Beispiels 11):

a) 5-(3-Chlor-phenyl)-indan-1-on:

3 g (14.2 mmol) 5-Brom-indan-1-on werden mit 2.22 g (14.2 mmol) 3-Chlorphenylboronsäure und 3 g (28.3 mmol) Natriumcarbonat in einer Mischung aus 100 ml Toluol, 20 ml Ethanol und 20 ml Wasser suspendiert. Unter Argonatmosphäre werden 160 mg (7.1 mmol) Palladium-II-acetat und 373 mg (14.2 mmol) Triphenylphosphin zugegeben. Die Mischung wird 3 h unter Rückfluß erhitzt, danach wird der Ethanolanteil des Lösemittelgemisches im Vakuum entfernt. Es werden 40 ml 0.5 N Natronlauge zugesetzt und das Gemisch wird 10 min. bei Raumtemperatur gerührt. Der Niederschlag wird abgesaugt; das Filtrat wird mit 40 ml Wasser neutral gewaschen und mit einer konzentrierten Kochsalzlösung (3 x 40 ml) nachgewaschen, über Magnesiumsulfat getrocknet, im Vakuum eingeengt und an Kieselgel mit Toluol / Essigsäureethylester 20 / 1 chromatographisch gereinigt. Man erhält 5-(3-Chlor-phenyl)-indan-1-on mit dem Schmelzpunkt 113°C.

b) 2-Brom-5-(3-chlor-phenyl)-indan-1-on:

2.42 g (10 mmol) 5-(3-Chlor-phenyl)-indan-1-on werden in 30 ml Eisessig gelöst, mit 10µl einer 48%igen HBr-Lösung in Wasser versetzt und unter Rühren tropfenweise mit einer Lösung von 0.77 ml (15 mmol) Brom in 7 ml Eisessig versehen. Nach 3 h Rühren bei Raumtemperatur wird das Reaktionsgemisch in eine Mischung aus 100 g Eis mit 70 ml Wasser und 100 mg $NaHSO_3$ gegossen und gerührt. Die entstandene Suspension wird mit 200 ml Dichlormethan ausgeschüttelt, und die organische Phase anschließend mit Wasser (3 x 100 ml) gewaschen, über Magnesiumsulfat getrocknet, im Vakuum eingeengt und an Kieselgel mit Toluol / Essigsäureethylester 50 / 1 chromatographisch gereinigt. Man erhält, neben etwas 2,3-Dibrom-5-(3-chlor-phenyl)-indan-1-on, 2-Brom-5-(3-chlor-phenyl)-indan-1-on mit dem Schmelzpunkt 110°C.

c) 6-(3-Chlor-phenyl)-2-methyl-8H-indeno[1,2-d]thiazol:

321 mg 2-Brom-5-(3-chlor-phenyl)-indan-1-on werden mit 83 mg Thioacetamid in 10 ml trockenem Aceton gelöst und 5 h bei 0°Cgerührt. Der Niederschlag, bestehend aus 6-(3-Chlor phenyl)-2-methyl-8,8a-dihydroindeno [1,2-d]thiazol-3a-ol Hydrobromid wird abgesaugt, mit Aceton gewaschen, im Vakuum getrocknet und anschließend in 20 ml trockenem Methanol gelöst. Die Lösung steht 2 Wochen bei Raumtemperatur. Man stellt mit Triethylamin basisch, engt ein und reinigt an Kieselgel mit Essigsäureethylester / n-Heptan 1 /1. Man erhält 6-(3-Chlor-phenyl)-2-methyl-8H-indeno[1,2-d]thiazol mit dem Schmelzpunkt 111-112°C neben 6-(3-Chlor-phenyl)-3a-methoxy-2-methyl-8,8a-dihydro-3aH-indeno[1,2-d]thiazol mit dem Schmelzpunkt 80-82°C.

Ausführungsbeispiel 5:

2-Methyl-6-(2,2,2-trifluorethoxy)-indeno[1,2-d]thiazol Hydrobromid (Verbindung des Beispiels 13):

a) 5-(2,2,2-Trifiuorethoxy)-indan-1-on:

2,2 ml 2,2,2-Trifluorethanol werden zu einem rührenden Gemisch aus 3,5 g 5-Fluorindan-1-on, 20 ml wasserfreiem Dimethylformamid und 4,1 g wasserfreien und gemahlenen Kaliumkarbonat gegeben und 10 Stunden bei 80°C gerührt. Man destilliert das Lösungsmittel unter verminderten Druck ab, löst den Rückstand in Ethylacetat und wäscht die organische Phase mehrmals mit Wasser. Man erhält das Indanonderivat als bräunlich kristallinen Feststoff nach Chromatographie an Kieselgel mit einem Gemisch aus gleichen Teilen Ethylacetat und Toluol als Elutionsmittel. Schmelzpunkt 93 - 97 °C.

b) 2-Brom-5-(2,2,2-trifluorethoxy)-indan-1-on:

Diese Verbindung erhält man durch Umsetzung von 0,9 g 5-(2,2,2-Trifluorethoxy)-indan-1-on mit 0,2 ml Brom in 25 ml Essigester. Die Verbindung wird ohne weitere Reinigung weiter verwendet.

c) 2-Methy)-6-(2,2,2-trifluorethoxy)-indeno[1,2-d]thiazol Hydrobromid:

2-Brom-5-(2,2,2-trifluorethoxy)-indan-1-on wird mit einer äquivalenten Menge Thioacetamid in Aceton 5 h bei Raumtemperatur gerührt. Der Niederschlag, bestehend aus 2-Methyl-6-(2,2,2-trifluorethoxy)-8,8a-dihydroindeno [1,2-d]thiazol-3a-ol Hydrobromid, wird abgetrennt und in 15 ml Eisessig gekocht. Das Lösungsmittel wird unter vermindertem Druck abdestilliert und der Rückstand unter Diisopropylether zur Kristallisation gebracht. Farblose Kristalle, Schmelzpunkt 220-224°C.

Ausführungsbeispiel 6:

8-Brom-2-phenyl-4,5-dihydro-naphtho[1,2-d]thiazol (Verbindung des Beispiels 19):

0.3 g 2,7-Dibrom-3,4-dihydro-2H-naphthalin-1-on werden in 10 ml Ethanol gelöst, mit 140 mg Thiobenzamid versetzt und 5 h zum Rückfluß erhitzt. Die Reaktionsmischung wird im Vakuum eingeengt, der Rückstand in 10 ml 1 N Natronlauge suspendiert und 1 h bei Raumtemperatur gerührt. Die Suspension wird abgesaugt, gründlich mit Wasser gewaschen und im Vakuum getrocknet. Man erhält 8-Brom-2-phenyl-4,5-dihydro-naphtho[1,2-d]thiazol mit dem Schmelzpunkt 85°C.

Ausführungsbeispiel 7:

2,5,6-Trimethyl-8H-indeno[1,2-d]thiazol Hydrobromid (Verbindung des Beispiels 20):

5,6-Dimethyl-indan-1-on wird wie vorstehend für die anderen Indan-1-one beschrieben in das 2-Brom-5,6-dimethyl-indan-1-on überführt. Dieses wird mit einer äquivalenten Menge Thioacetamid in Aceton zur Reaktion gebracht. Der Niederschlag, bestehend aus dem Hydrobromid des 2,5,6-Trimethyl-8,8a-dihydro-indeno[1,2-d]thiazol-3a-ols, wird in Eisessig erhitzt und liefert nach dem Entfernen des Lösemittels und Behandlung mit Diisopropylether 2,5,6-Trimethyl-8H-indeno[1,2-d]thiazol Hydrobromid mit dem Schmelzpunkt 290°C.

## Patentansprüche

1. Verwendung der Verbindungen der Formel I,

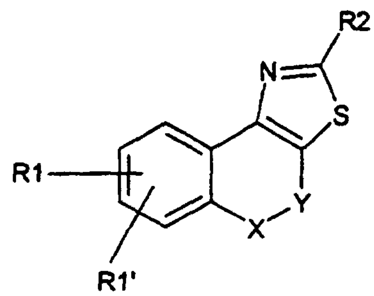

worin bedeuten

Y $CH_2$, $CH_2CH_2$;

X eine direkte Bindung, $CH_2$, O, NR3 oder S;

R1, R1' unabhängig voneinander H, F, Cl, Br, J, $CF_3$, CN, COOH, COO($C_1$-$C_6$)Alkyl, $CONH_2$, CONH($C_1$-$C_6$) Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, $OCF_3$, O-($C_2$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, CN, OC(O)$CH_3$, OC(O)H, O-$CH_2$-Ph, $NH_2$, NH-CO-$CH_3$ oder N($COOCH_2Ph$)$_2$ ersetzt sein kann;
$SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Phenyl, $SO_2$-($C_1$-$C_6$) -Alkyl, SO-($CH_2$)$_n$ -Phenyl, $SO_2$-($CH_2$)$_n$-Phenyl, wobei n = 0 - 6 sein kann und der Phenylrest bis zu zweifach mit F, Cl, Br, OH, $CF_3$, $NO_2$, CN, $OCF_3$, O-($C_1$-$C_6$)-Alkyl, ($C_1$-$C_6$)-Alkyl, $NH_2$ substituiert sein kann; $NH_2$, NH-($C_1$-$C_6$)-Alkyl, N(($C_1$-$C_6$)-Alkyl)$_2$, NH($C_1$-$C_7$)-Acyl, Phenyl, Biphenylyl, O-($CH_2$)$_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;

R2 ($C_1$-$C_8$)-Alkyl, ($C_3$-$C_7$)-Cycloalkyl, ($C_2$-$C_6$)-Alkenyl, ($C_2$-$C_6$)-Alkinyl, C(CN)=C($CH_3$)$_2$, C(O)$OCH_2CH_3$, $CH_2$-O-C(O)-C($CH_3$)$_3$, ($C_4$-$C_7$)-Cycloalkenyl, wobei in den Alkylresten ein, mehrere, oder alle Wasserstoff(e) durch Fluor ersetzt sein können oder ein Wasserstoff durch OH, CN, oder O-($C_1$-$C_4$)-Alkyl ersetzt sein kann;
($CH_2$)$_n$-NR6R7, wobei n = 1-6 sein kann und R6 und R7 unabhängig voneinander H, ($C_1$-$C_6$)-Alkyl, ($C_3$-$C_6$)-Cycloalkyl, CO-($C_1$-$C_6$)-Alkyl, CHO oder CO-Phenyl sein können, oder -NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
($CH_2$)$_n$-Aryl, wobei n = 0 - 6 sein kann und Aryl Phenyl, Biphenylyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3-oder 5-Isoxazolyl, 2- oder 3-Pyrrolyl, 2- oder 3-Pyridazinyl, 2-, 4- oder 5-Pyrimidinyl, 2-Pyrazinyl, (1,3,5-Triazinyl)-2, 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und der Arylrest oder Heteroarylrest bis zu zweifach mit F, Cl, Br, OH, $CF_3$, $NO_2$, CN, $OCF_3$, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]$_2$, CONH($C_3$-$C_6$)Cycloalkyl, $NH_2$, NH-CO-($C_1$-$C_6$)-Alkyl, NH-CO-Phenyl, Pyrrolidin-1-yl, Morpholin-1-yl, Piperidin-1-yl, Piperazin-1-yl, 4-Methyl-piperazin-1-yl, ($CH_2$)$_n$-Phenyl, O-($CH_2$)$_n$-Phenyl, S-($CH_2$)$_n$-Phenyl, $SO_2$-($CH_2$)$_n$-Phenyl, wobei n = 0-3, substituiert sein kann;

R3 H, ($C_1$-$C_6$)-Alkyl; CO-($C_1$-$C_5$)-Alkyl, CO-($C_3$-$C_6$)-cycloalkyl; $SO_2$-phenyl, p-Toluolsulfonyl;

sowie deren physiologisch verträgliche Salze zur Herstellung eines Medikaments zur Prophylaxe oder Behandlung von Obesitas.

**2.** Verwendung der Verbindungen der Formel I, gemäß Anspruch 1, **dadurch gekennzeichnet, daß** darin bedeuten

Y $CH_2$;

X eine direkte Bindung, $CH_2$;

R1, R1' unabhängig voneinander H, F, Cl, Br, J, $CF_3$, CN, COOH, COO($C_1$-$C_6$)Alkyl, $CONH_2$, CONH($C_1$-$C_6$)Alkyl, CON[($C_1$-$C_6$)Alkyl]$_2$, ($C_1$-$C_6$)-Alkyl, ($C_2$-$C_6$)-Alkenyl, ($C_2$-$C_6$)-Alkinyl, $OCF_3$, O-($C_2$-$C_6$)-Alkyl, wobei in den Alkyl-, Alkenyl- und Alkinylresten ein, mehrere, oder alle Wasserstoff(e) durch Fluor ersetzt sein können, oder ein Wasserstoff durch OH, CN, $NH_2$, ersetzt sein kann;
$NH_2$, NH-($C_1$-$C_6$)-Alkyl, N(($C_1$-$C_6$-Alkyl)$_2$, Phenyl, O-$(CH_2)_n$-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

R2 ($C_1$-$C_8$)-Alkyl, ($C_3$-$C_7$)-Cycloalkyl, ($C_2$-$C_6$)-Alkenyl, ($C_2$-$C_6$)-Alkiny), C(CN)=C$(CH_3)_2$, C(O)$OCH_2CH_3$, $CH_2$-O-C(O)-C$(CH_3)_3$, ($C_4$-$C_7$)-Cycloalkenyl, wobei in den Alkylresten ein, mehrere, oder alle Wasserstoff(e) durch Fluor ersetzt sein können oder ein Wasserstoff durch OH, CN, oder O-($C_1$-$C_4$)-Alkyl ersetzt sein kann;
$(CH_2)_n$-NR6R7, wobei n = 1-6 sein kann und R6 und R7 unabhängig voneinander H, ($C_1$-$C_6$)-Alkyl, ($C_3$-$C_6$)-Cycloalkyl, CO-($C_1$-$C_6$)-Alkyl, CHO oder CO-Phenyl sein können;
$(CH_2)_n$-Aryl, wobei n = 0 - 6 sein kann und Aryl Phenyl, Biphenylyl, 1- oder 2-Naphthyl, 2-, 3- oder 4-Pyridyl, Benzothiazol-2-yl, Indol-3-yl, Indol-5-yl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl, $CONH_2$;

sowie deren physiologisch verträgliche Salze zur Herstellung eines Medikaments zur Prophylaxe oder Behandlung von Obesitas.

**3.** Verwendung der Verbindungen der Formel I, gemäß Anspruch 1 oder 2, **dadurch gekennzeichnet, daß** darin bedeuten

Y -$CH_2$-;

X -$CH_2$-, eine direkte Bindung;

R1 Cl, Br, ($C_1$-$C_6$,)-Alkyl, $OCF_3$, O-($C_2$-$C_6$)-Alkyl, wobei in den Alkylresten ein, mehrere, oder alle Wasserstoff (e) durch Fluor ersetzt sein können;
Phenyl, das bis zu 3-fach substituiert sein kann mit F, Cl, Br, OH, ($C_1$-$C_6$)-Alkyl;

R1' H oder R1;

R2 ($C_1$-$C_6$)-Alkyl, ($C_1$-$C_6$)-Alkyl-CN, C(CN)=C$(CH_3)_2$, C(O)$OCH_2CH_3$, $CH_2$-O-C(O)-C$(CH_3)_3$;
$(CH_2)_n$-Aryl, wobei n = 0 - 2 sein kann und Aryl gleich Phenyl, 2, 3- oder 4-Pyridyl, Benzothiazol-2-yl, Indol-3-yl, Indol-5-yl sein kann und der Arylrest oder Heteroarylrest bis zu zweifach mit F, Cl, Br, OH, $CF_3$, $NO_2$; CN, $OCF_3$, ($C_1$-$C_6$)-Alkyl, O-($C_1$-$C_6$)-Alkyl, substituiert sein kann;

sowie deren physiologisch verträgliche Salze zur Herstellung eines Medikaments zur Prophylaxe oder Behandlung von Obesitas.

**4.** Verwendung der Verbindungen gemäß einem oder mehreren der Ansprüche 1 bis 3 in Kombination mit einem oder mehreren anorektischen Wirkstoffen zur Herstellung eines Medikaments zur Prophylaxe oder Behandlung von Obesitas.

## Claims

1. The use of compounds of the formula I

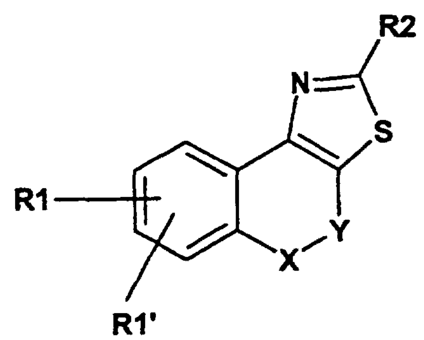

in which

Y is $CH_2$, $CH_2$-$CH_2$;

X is a direct linkage, $CH_2$, O, NR3 or S;

R1, R1' are, independently of one another H, F, Cl, Br, I, $CF_3$, CN, COOH, COO($C_1$-$C_6$)alkyl, $CONH_2$, CONH ($C_1$-$C_6$)alkyl, CON[($C_1$-$C_6$)alkyl]$_2$, ($C_1$-$C_6$)-alkyl, ($C_2$-$C_6$)-alkenyl, ($C_2$-$C_6$)-alkynyl, $OCF_3$, O-($C_2$-$C_6$)-alkyl, where one, more than one or all hydrogen(s) in the alkyl, alkenyl and alkynyl radicals can be replaced by fluorine, or one hydrogen can be replaced by OH, CN, OC(O)$CH_3$, OC(O)H, O-$CH_2$-Ph, $NH_2$, NH-CO-$CH_3$ or N(COO$CH_2$Ph)$_2$;
$SO_2$-$NH_2$, $SO_2$NH($C_1$-$C_6$)-alkyl, $SO_2$N[($C_1$-$C_6$)-alkyl]$_2$, S-($C_1$-$C_6$)-alkyl, S-($CH_2$)$_n$-phenyl, $SO_2$-($C_1$-$C_6$)-alkyl, SO-($CH_2$)$_n$-phenyl, $SO_2$-($CH_2$)$_n$-phenyl, where n can be 0-6 and the phenyl radical can be substituted up to twice by F, Cl, Br, OH, $CF_3$, $NO_2$, CN, $OCF_3$, O-($C_1$-$C_6$)-alkyl, ($C_1$-$C_6$)-alkyl, $NH_2$; $NH_2$, NH-($C_1$-$C_6$)-alkyl, N(($C_1$-$C_6$)-alkyl)$_2$, NH($C_1$-$C_7$)-acyl, phenyl, biphenylyl, O-($CH_2$)$_n$-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted up to 3 times by F, Cl, Br, 1, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;
1,2,3-triazol-5-yl, it being possible for the triazole ring to be substituted in position 1, 2 or 3 by methyl or benzyl;
tetrazol-5-yl, it being possible for the tetrazole ring to be substituted in position 1 or 2 by methyl or benzyl;

R2 is ($C_1$-$C_8$)-alkyl, ($C_3$-$C_7$)-cycloalkyl, ($C_2$-$C_6$)-alkenyl, ($C_2$-$C_6$)-alkynyl, C(CN)=C($CH_3$)$_2$, C(O)O$CH_2$$CH_3$, $CH_2$-O-C(O)-C($CH_3$)$_3$, ($C_4$-$C_7$)cycloalkenyl, where one, more than one or all hydrogen(s) in the alkyl radicals can be replaced by fluorine, or one hydrogen can be replaced by OH, CN or O-($C_1$-$C_4$)-alkyl;
($CH_2$)$_n$-NR6R7, where n can be 1-6, and R6 and R7 can be, independently of one another, H, ($C_1$-$C_6$)-alkyl, ($C_3$-$C_6$)-cycloalkyl, CO-($C_1$-$C_6$)-alkyl, CHO or CO-phenyl, or -NR6R7 is a ring such as pyrrolidine, piperidine, morpholine, piperazine, N-4-methylpiperazin-1-yl, N-4-benzyipiperazin-1-yl, phthalimidyl;
($CH_2$)$_n$-aryl, where n can be 0-6, and aryl can be phenyl, biphenylyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 1,3,5-triazin-2-yl, 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl, and the aryl radical or heteroaryl radical can be substituted up to twice by F, Cl, Br, OH, $CF_3$, $NO_2$, CN, $OCF_3$, O-($C_1$-$C_3$)-alkyl, S-($C_1$-$C_6$)-alkyl, SO-($C_1$-$C_6$)-alkyl, $SO_2$-($C_1$-$C_6$)-alkyl, ($C_1$-$C_6$)-alkyl, ($C_3$-$C_6$)-cycloalkyl, COOH, COO($C_1$-$C_6$)alkyl, COO($C_3$-$C_6$)cycloalkyl, $CONH_2$, CONH($C_1$-$C_6$)alkyl, CON[($C_1$-$C_6$)alkyl]$_2$, CONH($C_3$-$C_6$)cycloalkyl, $NH_2$, NH-CO-($C_1$-$C_6$)-alkyl, NH-CO-phenyl, pyrrolidin-1-yl, morpholin-1-yl, piperidin-1-yl, piperazin-1-yl, 4-methylpiperazin-1-yl, ($CH_2$)$_n$-phenyl, O-($CH_2$)$_n$-phenyl, S-($CH_2$)$_n$-phenyl, $SO_2$-($CH_2$)$_n$-phenyl, where n = 0-3;

R3 is H, ($C_1$-$C_6$)-alkyl; CO-($C_1$-$C_5$)-alkyl, CO-($C_3$-$C_6$)-cycloalkyl; $SO_2$-phenyl, p-toluenesulfonyl;

and their physiologically tolerated salts for producing a medicine for the prophylaxis or treatment of obesity.

**2.** The use of compounds of the formula I as claimed in claim 1, wherein the meanings are as follows

Y $CH_2$;

X a direct linkage, $CH_2$;

R1, R1' independently of one another, H, F, Cl, Br, I, $CF_3$, CN, COOH, COO($C_1$-$C_6$)alkyl, $CONH_2$, CONH($C_1$-$C_6$) alkyl, CON[($C_1$-$C_6$)alkyl]$_2$, ($C_1$-$C_6$)-alkyl, ($C_2$-$C_6$)-alkenyl, ($C_2$-$C_6$)-alkynyl, $OCF_3$, O-($C_2$-$C_6$)-alkyl, where one, more than one or all hydrogen(s) in the alkyl, alkenyl and alkynyl radicals can be replaced by fluorine, or one hydrogen can be replaced by OH, CN, $NH_2$;
$NH_2$, NH-($C_1$-$C_6$)-alkyl, N(($C_1$-$C_6$)-alkyl)$_2$, phenyl, O-$(CH_2)_n$-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted one to 3 times by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$)-alkyl, $CONH_2$;

R2 ($C_1$-$C_8$)-alkyl, ($C_3$-$C_7$)-cycloalkyl, ($C_2$-$C_6$)-alkenyl, ($C_2$-$C_6$)-alkynyl, C(CN)=C$(CH_3)_2$, C(O)$OCH_2CH_3$, $CH_2$-O-C(O)-C(CH3)3,
($C_4$-$C_7$)-cycloalkenyl, where one, more than one or all hydrogen(s) in the alkyl radicals can be replaced by fluorine, or one hydrogen can be replaced by OH, CN, or O-($C_1$-$C_4$)-alkyl; $(CH_2)_n$-NR6R7, where n can be 1-6, and R6 and R7 can be, independently of one another, H, ($C_1$-$C_6$)-alkyl, ($C_3$-$C_6$)-cycloalkyl, CO-($C_1$-$C_6$)-alkyl, CHO or CO-phenyl;
$(CH_2)_n$-aryl, where n can be 0-6, and aryl can be phenyl, biphenylyl, 1- or 2-naphthyl, 2-, 3- or 4-pyridyl, benzothiazol-2-yl, indol-3-yl, indol-5-yl, 2- or 3-furanyl or 2- or 3-thienyl, it being possible for the phenyl, biphenylyl, naphthyl, pyridyl, furanyl or thienyl rings each to be substituted one to 3 times by F, Cl, Br, I, OH, $CF_3$, $NO_2$, CN, $OCF_3$, O-($C_1$-$C_6$)-alkyl, ($C_1$-$C_6$)-alkyl, $NH_2$, NH($C_1$-$C_6$)-alkyl, N(($C_1$-$C_6$)-alkyl)$_2$, $SO_2$-$CH_3$, COOH, COO-($C_1$-$C_6$-alkyl, $CONH_2$;

and their physiologically tolerated salts for producing a medicine for the prophylaxis or treatment of obesity.

**3.** The use of compounds of the formula I as claimed in claim 1 or 2, wherein the meanings are as follows

Y -$CH_2$-;

X -$CH_2$-, a direct linkage;

R1 Cl, Br, ($C_1$-$C_6$)-alkyl, $OCF_3$, O-($C_2$-$C_6$)-alkyl, where one, more than one or all hydrogen(s) in the alkyl radicals can be replaced by fluorine;
phenyl which can be substituted up to 3 times by F, Cl, Br, OH, ($C_1$-$C_6$)-alkyl;

R1' H or R1;

R2 ($C_1$-$C_6$)-alkyl, ($C_1$-$C_6$)-alkyl-CN, C(CN)=C$(CH_3)_2$,
C(O)$OCH_2CH_3$, $CH_2$-O-C(O)-C$(CH_3)_3$;
$(CH_2)_n$-aryl, where n can be 0-2, and aryl can be equal to phenyl, 2-, 3- or 4-pyridyl, benzothiazol-2-yl, indol-3-yl, indol-5-yl and the aryl radical or heteroaryl radical can be substituted up to twice by F, Cl, Br, OH, $CF_3$, $NO_2$, CN, $OCF_3$, ($C_1$-$C_6$)-alkyl, O-($C_1$-$C_6$)-alkyl;

and their physiologically tolerated salts for producing a medicine for the prophylaxis or treatment of obesity.

**4.** The use of the compounds as claimed in one or more of claims 1 to 3 in combination with one or more anorectic active ingredients for producing a medicine for the prophylaxis or treatment of obesity.

## Revendications

1. Utilisation des composés de formule I

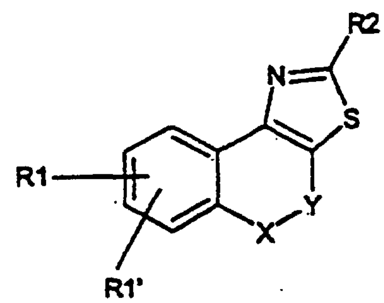

dans laquelle

Y représente un groupe $CH_2$, $CH_2$-$CH_2$ ;

X représente une liaison directe, un groupe $CH_2$, O, NR3 ou S ;

R1, R1' indépendamment l'un de l'autre, représentent un atome d'hydrogène, de F, de Cl, de Br, d'I, un groupe $CF_3$, CN, COOH, COO-alkyle en $C_1$-$C_6$, $CONH_2$, CONH-alkyle en $C_1$-$C_6$, CON (alkyle en $C_1$-$C_6$)$_2$, alkyle en $C_1$-$C_6$, alcényle en $C_2$-$C_6$, alcynyle en $C_2$-$C_6$, $OCF_3$, O-alkyle en $C_2$-$C_6$, dans les restes alkyle, alcényle et alcynyle un, plusieurs ou tous les atomes d'hydrogène pouvant être remplacés par des atomes de fluor, ou un atome d'hydrogène peut être remplacé par un groupe OH, CN, OC(O)$CH_3$, OC (O)H, O-$CH_2$-Ph, $NH_2$, NH-CO-$CH_3$ ou N(COO$CH_2$Ph)$_2$ ; $SO_2$-$NH_2$, $SO_2$NH-alkyle en $C_1$-$C_6$, $SO_2$N-(alkyle en $C_1$-$C_6$)$_2$, S-alkyle en $C_1$-$C_6$, S- $(CH_2)_n$-phényle, $SO_2$-alkyle en $C_1$-$C_6$, SO-$(CH_2)_n$-phényle, $SO_2$-$(CH_2)_n$-phényle, où n peut être = 0-6 et le reste phényle peut être substitué jusqu'à deux fois par des substituants F, Cl, Br, OH, $CF_3$, $NO_2$, CN, $OCF_3$, O-alkyle en $C_1$-$C_6$, alkyle en $C_1$-$C_6$, $NH_2$ ; $NH_2$, NH-alkyle en $C_1$-$C_6$, N(alkyle en $C_1$-$C_6$)$_2$, NH-acyle en $C_1$-$C_7$, phényle, biphénylyle, O-$(CH_2)_n$-phényle, n pouvant être = 0-6, 1- ou 2-naphtyle, 2-, 3- ou 4-pyridyle, 2- ou 3-furanyle ou 2- ou 3-thiényle, les cycles phényle, biphénylyle, naphtyle, pyridyle, furanyle ou thiényle chacun pouvant être substitué une à trois fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyl en $C_1$-$C_6$)$_2$, $SO_2CH_3$, COOH, COO-alkyle en $C_1$-$C_6$, $CONH_2$ ;
1,2,3-triazol-5-yle, le cycle triazole pouvant être substitué en position 1, 2 ou 3 par un substituant méthyle ou benzyle ;
tétrazol-5-yle, le cycle tétrazole pouvant être substitué en position 1 ou 2 par un substituant méthyle ou benzyle ;

R2 représente un groupe alkyle en $C_1$-$C_8$, cycloalkyle en $C_3$-$C_7$, alcényle en $C_2$-$C_6$, alcynyle en $C_2$-$C_6$, C (CN)=C($CH_3$)$_2$, C(O)O$CH_2CH_3$, $CH_2$-O-C(O)-C($CH_3$)$_3$,
cycloalcényle en $C_4$-$C_7$, dans les restes alkyle un, plusieurs ou tous les atomes d'hydrogène pouvant être remplacés par des atomes de fluor ou un atome d'hydrogène peut être remplacé par un groupe OH, CN ou O-alkyle en $C_1$-$C_4$ ;
$(CH_2)_n$-NR6R7, n pouvant être = 1-6 et R6 et R7, indépendamment l'un de l'autre, peuvent représenter un atome d'hydrogène, un groupe alkyle en $C_1$-$C_6$, cycloalkyle en $C_3$-$C_6$, CO-alkyle en $C_1$-$C_6$, CHO ou CO-phényle, ou -NR6R7 représente un cycle comme pyrrolidine, pipéridine, morpholine, pipérazine, N-4-méthyl-pipérazin-1-yle, N-4-benzyl-pipérazin-1-yle, phtalimidyle ;
$(CH_2)_n$-aryle, n pouvant être = 0-6 et aryle peut représenter un groupe phényle, biphénylyle, 1- ou 2-naphtyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3- ou 5-isoxazolyle, 2- ou 3-pyrrolyle, 2- ou 3-pyridazinyle, 2-, 4- ou 5-pyrimidinyle, 2-pyrazinyle, (1,3,5-triazinyl)-2,2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthylimidazol-2-, 4- ou 5-yle et le reste aryle ou hétéroaryle peut être substitué jusqu'à deux fois par des substituants F, Cl, Br, OH, $CF_3$, $NO_2$, CN, $OCF_3$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$, CON (alkyle en $C_1$-$C_6$)$_2$, CONH-cycloalkyle en $C_3$-$C_6$, $NH_2$, NH-CO-alkyle en $C_1$-$C_6$, NH-CO-phényle, pyrrolidin-1-yle, morpholin-1-yle, pipéridin-1-yle, pipérazin-1-yle, 4-méthylpipérazin-1-yle, $(CH_2)_n$-phényle, O-$(CH_2)_n$-phényle, S-$(CH_2)_n$-phényle, $SO_2$-$(CH_2)_n$-phényle, n = 0-3 ;

R3 représente un atome d'hydrogène, un groupe alkyle en $C_1$-$C_6$; CO-alkyle en $C_1$-$C_5$; CO-cycloalkyle en $C_3$-$C_6$ ; $SO_2$-phényle, p-toluènesulfonyle ;

ainsi que leurs sels physiologiquement tolérés, pour la préparation d'un médicament pour la prévention ou le traitement de l'obésité.

2. Utilisation des composés de formule I, selon la revendication 1, **caractérisés en ce que**

Y représente $CH_2$,
X représente une liaison directe, un groupe $CH_2$ ;
R1, R1' indépendamment l'un de l'autre, représentent un atome d'hydrogène, de F, de Cl, de Br, d'I, un groupe $CF_3$, CN, COOH, COO-alkyle en $C_1$-$C_6$, $CONH_2$, CONH-alkyle en $C_1$-$C_6$, CON (alkyle en $C_1$-$C_6$)$_2$, alkyle en $C_1$-$C_6$, alcényle en $C_2$-$C_6$, alcynyle en $C_2$-$C_6$, $OCF_3$, O-alkyle en $C_2$-$C_6$, dans les restes alkyle, alcényle et alcynyle un, plusieurs ou tous les atomes d'hydrogène pouvant être remplacés par des atomes de fluor, ou un atome d'hydrogène peut être remplacé par un groupe OH, CN, $NH_2$ ;
$NH_2$, NH-alkyle en $C_1$-$C_6$, N (alkyle en $C_1$-$C_6$)$_2$, phényle, O-$(CH_2)_n$-phényle, n pouvant être = 0-6, 1- ou 2-naphtyle, 2-, 3- ou 4-pyridyle, 2- ou 3-furanyle ou 2- ou 3-thiényle, les cycles phényle, biphénylyle, naphtyle, pyridyle, furanyle ou thiényle chacun pouvant être substitué une à trois fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N(alkyl en $C_1$-$C_6$)$_2$, $SO_2$-$CH_3$, COOH, COO-alkyle en $C_1$-$C_6$, $CONH_2$ ;
R2 représente un groupe alkyle en $C_1$-$C_8$, cycloalkyle en $C_3$-$C_7$, alcényle en $C_2$-$C_6$, alcynyle en $C_2$-$C_6$, C (CN) =$C(CH_3)_2$, $C(O)OCH_2CH_3$, $CH_2$-O-C(O)-$C(CH_3)_3$,
cycloalcényle en $C_4$-$C_7$, dans les restes alkyle un, plusieurs ou tous les atomes d'hydrogène pouvant être remplacés par des atomes de fluor, ou un atome d'hydrogène peut être remplacé par un groupe OH, CN ou O-alkyle en $C_1$-$C_4$ ;
$(CH_2)_n$-NR6R7, n pouvant être = 1-6 et R6 et R7, indépendamment l'un de l'autre, représentent un atome d'hydrogène, un groupe alkyle en $C_1$-$C_6$, cycloalkyle en $C_3$-$C_6$, CO-alkyle en $C_1$-$C_6$, CHO ou CO-phényle ;
$(CH_2)_n$-aryle, n pouvant être = 0-6 et aryle représente un groupe phényle, biphénylyle, 1- ou 2-naphtyle, 2-, 3- ou 4-pyridyle, benzothiazol-2-yle, indol-3-yle, indol-5-yle, 2- ou 3-furanyle ou 2- ou 3-thiényle, les cycles phényle, biphénylyle, naphtyle, pyridyle, furanyle ou thiényle chacun pouvant être substitué une à trois fois par des substituants F, Cl, Br, I, OH, $CF_3$, $NO_2$, CN, $OCF_3$, O-alkyle en $C_1$-$C_6$, alkyle en $C_1$-$C_6$, $NH_2$, NH-alkyle en $C_1$-$C_6$, N-(alkyle en $C_1$-$C_6$)$_2$, $SO_2$-$CH_3$, COOH, COO-alkyle en $C_1$-$C_6$, $CONH_2$ ;

ainsi que leurs sels physiologiquement tolérés, pour la préparation d'un médicament pour la prévention ou le traitement de l'obésité.

3. Utilisation des composés de formule I selon la revendication 1 ou 2, **caractérisés en ce que**

Y représente un groupe -$CH_2$- ;
X représente un groupe -$CH_2$-, une liaison directe ;
R1 représente un atome de Cl, de Br, un groupe alkyle en $C_1$-$C_6$, $OCF_3$, O-alkyle en $C_1$-$C_6$, dans les restes alkyle un, plusieurs ou tous les atomes d'hydrogène pouvant être remplacés par des atomes de fluor ;
phényle qui peut être substitué jusqu'à trois fois par des substituants F, Cl, Br, OH, alkyle en $C_1$-$C_6$ ;
R1' représente un atome d'hydrogène ou R1 ;
R2 représente un groupe alkyle en $C_1$-$C_6$, (alkyl en $C_1$-$C_6$) CN, C(CN) =$C(CH_3)_2$, $C(O)OCH_2CH_3$, $CH_2$-O-C(O) -$C(CH_3)_3$,
$(CH_2)_n$-aryle, n pouvant être = 0-2 et aryle peut représenter phényle, 2-, 3- ou 4-pyridyle, benzothiazol-2-yle, indol-3-yle, indol-5-yle et le reste aryle ou le reste hétéroaryle pouvant être substitué jusqu'à deux fois par des substituants F, Cl, Br, OH, $CF_3$, $NO_2$, CN, $OCF_3$, alkyle en $C_1$-$C_6$, O-alkyle en $C_1$-$C_6$ ;

ainsi que leurs sels physiologiquement tolérés, pour la préparation d'un médicament pour la prévention ou le traitement de l'obésité.

4. Utilisation des composés selon une ou plusieurs des revendications 1 à 3 en combinaison avec un ou plusieurs principes actifs anorexigènes pour la préparation d'un médicament pour la prévention ou le traitement de l'obésité.